# EUROPEAN PATENT APPLICATION

(11) **EP 4 397 650 A1**
(43) Date of publication of application: **10.07.2024**
(21) Application number: 22864681.6
(22) Date of filing: 01.09.2022
(51) Int. Cl.: C07C 29/88, C07C 31/20, G01N 30/88

(54) **METHOD FOR PRODUCING PRODUCT 1,3-BUTYLENE GLYCOL**

(30) Priority: 02.09.2021 JP 2021143519
(71) Applicant: KH Neochem Co., Ltd., Tokyo 103-0022 (JP)
(72) Inventor: KURAMOTO, Kouji, Yokkaichi-shi, Mie 510-8502 (JP); TACHIBANA, Yuki, Kawasaki-shi, Kanagawa 212-0032 (JP); TANAKA, Hirotaka, Kawasaki-shi, Kanagawa 212-0032 (JP)
(74) Representative: V.O.
(86) International application number: PCT/JP2022/032908
(87) International publication number: WO 2023/033099

(57) **Abstract**

A method for producing a product 1,3-butylene glycol, including a step of subjecting crude 1,3-butylene glycol to hydrogenation treatment using a Pd/C catalyst.

## Description

### Technical Field

The present invention relates to a production method of a product 1,3-butylene glycol that is useful as a raw material of synthetic resins, a raw material of surfactants, a solvent, an antifreeze, a cosmetic raw material or the like.

### Background Art

1,3-Butylene glycol is a viscous, colorless and transparent liquid having a boiling point of 208°C and a low odor and has excellent chemical stability. Therefore, 1,3-butylene glycol is used as a raw material of a variety of synthetic resins and surfactants. 1,3-Butylene glycol is also in use as a material for cosmetics, moisture absorbers, high-boiling point solvents and antifreezes due to its excellent moisture absorption characteristic, low volatility and low toxicity. Particularly, in recent years, the demand for 1,3-butylene glycol has been significantly growing in the cosmetic industry since 1,3-butylene glycol with low toxicity and irritating properties has excellent properties as a moisturizer.

Patent Literature 1 discloses 1,3-butylene glycol having a weak odor. Furthermore, as a method for obtaining 1,3-butylene glycol having a weak odor, a production method of 1,3-butylene glycol in which crude 1,3-butylene glycol is purified by a hydrogenation treatment is disclosed.

### Citation List

### Patent Literature

Patent Literature 1: Japanese Translation of PCT International Application Publication No. 2020-512351

### Summary of Invention

### Technical Problem

However, in the method described in Patent Literature 1, there is a problem in that it is difficult to sufficiently reduce the odor and thus, when 1,3-butylene glycol is stored for a long period of time, a slight odor is generated due to a change over time.

Additionally, in the cosmetics field, there are cases where 1,3-butylene glycol is directly applied to the skin during use, but 1,3-butylene glycol that is obtained by the method described in Patent Literature 1 has a problem in that the skin sensitization has not been sufficiently reduced.

In consideration of the above-described circumstances, an objective of the present invention is to provide a production method of a product 1,3-butylene glycol having not only a sufficiently reduced odor but also reduced skin sensitization.

### Solution to Problem

As a result of intensive studies, the present inventors have found that the above-described problems can be solved by subjecting crude 1,3-butylene glycol to hydrogenation treatment using a Pd/C catalyst and have completed the present invention.

More specifically, the present invention is as described below.
[1] A method for producing a product 1,3-butylene glycol, comprising: a step of subjecting crude 1,3-butylene glycol to hydrogenation treatment using a Pd/C catalyst.
[2] The method for producing the product 1,3-butylene glycol according to [1], wherein in the step of subjecting crude 1,3-butylene glycol to hydrogenation treatment, a reaction temperature is 50°C to 120°C.
[3] The production method of the product 1,3-butylene glycol according to [1] or [2], wherein in the step of subjecting crude 1,3-butylene glycol to hydrogenation treatment, a content of 6-hydroxy-2-hexanone in the crude 1,3-butylene glycol is reduced.
[4] A product 1,3-butylene glycol, wherein an area percentage of a peak of 1,3-butylene glycol in a gas chromatography analysis under the following conditions is 99.5% or more, and a weight fraction of a dinitrophenylhydrazine derivative of 6-hydroxy-2-hexanone in an HPLC analysis under the following conditions is 18.0 ppm or less,
   wherein the conditions of the gas chromatography analysis are as follows:
      analysis column: a column in which polyethylene glycol is used as a stationary phase (length of 30 m × inner diameter of 0.25 mm × film thickness of 0.25 µm),
      temperature rising conditions: a temperature is raised from 80°C up to 230°C at 5 °C/minute and then retained at 230°C for 10 minutes,
      sample introduction temperature: 250°C,
      carrier gas: nitrogen,
      gas flow rate in column: 0.5 mL/minute,
      detector and detection temperature: flame ionization detector (FID), 250°C,
      control mode: constant flow
      split ratio: 50:1,
      sample injection condition: 1 µL, and
   wherein the conditions of the HPLC analysis are as follows:
      the sample preparation is as follows:
      1000 µL of a solution of 2,4-dinitrophenylhydrazine which is extracted by adding 5 mL of acetonitrile to a cartridge packed with spherical silica gel coating 2,4-dinitrophenylhydrazine and 100 µL of 0.2 mol/L hydrochloric acid are added to 0.05 g of the product 1,3-butylene glycol and reacted at 45°C for two hours.

### (Analysis Conditions)

Measurement sample: a reaction liquid obtained by the sample preparation is diluted to 2 mL with a mobile phase that is used in HPLC, and the diluted liquid is used as a measurement sample,
Detector: UV-Vis detector,
Detection wavelength: 369 nm,
Analysis column: a column in which palmitamidopropyl group-modified silica gel (particle diameter: 5 µm, inner diameter × length = 4.6 mm × 25 cm, pore size: 100 Å, surface coating level: 2.7 µmol/m², surface area: 450 m²/g, metal impurity: less than 5 ppm, carbon content: 19.5%) is used as a stationary phase,
Column temperature: 40°C,
Mobile phase: acetonitrile/distilled water = 50/50 (volume ratio),
Mobile phase flow rate: 0.4 mL/min.,
Sample injection amount: 20 µL

### Advantageous Effects of Invention

The production method of the present invention makes it possible to provide a product 1,3-butylene glycol having not only a sufficiently reduced odor but also reduced skin sensitization.

### Brief Description of Drawing

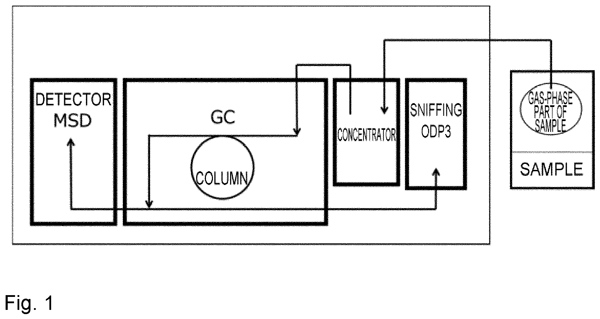

[Figure 1] Figure 1 is an example of a device that is used in a GC/MS-olfactometry measurement in the present invention.

### Description of Embodiments

Hereinafter, an embodiment of the present invention (hereinafter, referred to as "present embodiment") will be described in detail. The present invention is not limited to the following description and can be carried out after being modified in a variety of manners within the scope of the gist thereof.

In the present embodiment, 1,3-butylene glycol that is the final product will be also referred to as "product 1,3-butylene glycol", and 1,3-butylene glycol as a raw material or an intermediate before the final product is produced will be also referred to as "crude 1,3-butylene glycol".

In addition, in the present embodiment, there are cases where a component having a higher boiling point than 1,3-butylene glycol will be referred to as "high-boiling component" and a component having a lower boiling point than 1,3-butylene glycol will be referred to as "low-boiling component".

A method for producing a product 1,3-butylene glycol of the present embodiment includes a step of subjecting crude 1,3-butylene glycol to hydrogenation treatment using a Pd/C catalyst.

### [Method for Producing Product 1,3-Butylene Glycol] (Raw Material)

Crude 1,3-butylene glycol that is used as a raw material at the time of producing the product 1,3-butylene glycol in the present embodiment is not particularly limited, and examples thereof include 1,3-butylene glycol from which an odor is sensed or 1,3-butylene glycol the odor of which becomes stronger over time. Alternatively, examples thereof also include 1,3-butylene glycol having skin sensitization.

As the crude 1,3-butylene glycol as the raw material, the area percentage of the peak of 1,3-butylene glycol in a gas chromatography analysis under the specific conditions to be described below is preferably 99.5% or more, more preferably 99.6% or more and still more preferably 99.7% or more from the viewpoint of reducing the amount of an impurity contained in the product 1,3-butylene glycol. In addition, as the crude 1,3-butylene glycol, crude 1,3-butylene glycol for which the weight fraction of a dinitrophenylhydrazine derivative of 6-hydroxy-2-hexanone (hereinafter, also simply referred to as "peak weight fraction") exceeds 18.0 ppm in an HPLC analysis under the specific conditions to be described below is preferably used.

A production method of the crude 1,3-butylene glycol as the raw material is not particularly limited, and the crude 1,3-butylene glycol can be produced by, for example, a well-known method (refer to Japanese Patent Publication No. H3-80139, Japanese Patent Laid-Open No. 7-258129 and the like). In addition, any of crude 1,3-butylene glycol produced by a liquid phase hydrogen reduction method of acetaldol, crude 1,3-butylene glycol produced by a hydrolysis method of 1,3-butylene oxide, crude 1,3-butylene glycol produced by a fermentation method in which a microbe or a fungus is used, a mixture thereof and the like may also be used. Among these, a reaction product obtained by the liquid phase hydrogen reduction method of acetaldol is preferably used since there is a tendency that the effect of the present invention becomes more significant. In the liquid phase hydrogen reduction method of acetaldol, a low-boiling component such as acetaldehyde, butyraldehyde, crotonaldehyde or methyl vinyl ketone, which is considered to be an odor-causing substance, a condensate of the low-boiling component, an acetal of the low-boiling component and 1,3-butylene glycol, an acetal of the low-boiling component and ethanol or the like is generated as a byproduct, and it is difficult to sufficiently remove the low-boiling component or the like even by distillation. As the odor-causing substance, a substance that acts as an odor source, a substance that turns into an odor substance due to a change over time, a heating treatment, a chemical treatment or the like and the like are included.

The reaction product that is obtained by a hydrogen reduction method of acetaldol may also be used after alcohol such as ethanol, a salt, moisture or the like, which is a byproduct, is removed. A method for removing the above-described components is not limited, and a method such as distillation or adsorption can be used.

In addition, a substance obtained by further performing one or more well-known purification steps, for example, a step of adding an alkali metal compound (for example, sodium hydroxide, potassium hydroxide or the like) to a liquid from which ethanol or the like has been removed and then performing a heating treatment (refer to Japanese Patent No. 4559625 or the like) may also be used as the crude 1,3-butylene glycol. The crude 1,3-butylene glycol can also be procured as a commercially available product.

### (Hydrogenation Treatment)

The production method of a product 1,3-butylene glycol in the present embodiment includes a step of subjecting crude 1,3-butylene glycol to hydrogenation treatment using a Pd/C catalyst (hydrogenation treatment step). The hydrogenation treatment step is more specifically, for example, a treatment step in which hydrogen reduction is performed by circulating crude 1,3-butylene glycol and a hydrogen gas in a catalyst layer packed with a Pd/C catalyst.

It is presumed that, when a hydrogenation treatment is performed on crude 1,3-butylene glycol using a Pd/C catalyst, 6-hydroxy-2-hexanone, which is an odor-causing substance and a skin sensitization-causing substance, is hydrogenated or hydrocracked and, furthermore, methyl vinyl ketone, which is an odor-causing substance, is hydrogenated. However, the mechanism of the present invention is not limited to what has been described above.

The hydrogenation treatment step can be performed in any of a gas phase or a liquid phase, but is preferably performed in a liquid phase. In a case where the hydrogenation treatment step is performed in a liquid phase, since the reaction temperature can be set to a low temperature compared with that in a gas-phase reaction, it is possible to suppress the pyrolysis reaction of 1,3-butylene glycol. In the hydrogenation treatment step, a Pd/C catalyst is used as a catalyst. The Pd/C catalyst refers to a catalyst in which activated carbon is used as a carrier and palladium is dispersed and carried thereon. The carrying percentage of palladium that is dispersed and carried on activated carbon is not particularly limited, but is preferably 0.1% to 30%, more preferably 0.2% to 10% and particularly preferably 0.4% to 1%. In addition, the shape of the Pd/C catalyst is not particularly limited and may be a shape of a powder, a pellet or the like.

The reaction temperature in the hydrogenation treatment step is not particularly limited, but is preferably 50°C to 120°C, more preferably 60°C to 110°C and still more preferably 70°C to 100°C.

When the reaction temperature in the hydrogenation treatment step is 120°C or lower, there is a tendency that the pyrolysis reaction of 1,3-butylene glycol does not progress and an increase in a high-boiling component is suppressed, and, when the reaction temperature is 60°C or higher, the progress of the hydrogenation or hydrocracking of 6-hydroxy-2-hexanone tends to be accelerated.

The hydrogen pressure in the hydrogenation treatment step is not particularly limited, but is preferably 0.4 to 1.0 MPa, more preferably 0.5 to 0.9 MPa and still more preferably 0.6 to 0.8 MPa.

When the hydrogen pressure in the hydrogenation treatment step is 1.0 MPa or lower, the device cost tends to be reduced since a sufficiently strong and thick-walled device or the like is not required as a highpressure gas facility, and when the hydrogen pressure is 0.4 MPa or higher, the progress of the hydrogenation or hydrocracking of 6-hydroxy-2-hexanone tends to be accelerated.

In the production method of a product 1,3-butylene glycol in the present embodiment, it is preferable to reduce the content of 6-hydroxy-2-hexanone in the crude 1,3-butylene glycol by the hydrogenation step from the viewpoint of further reducing the odor and the skin sensitization.

The production method of a product 1,3-butylene glycol in the present embodiment is not particularly limited as long as the hydrogenation treatment step is included, may further include, aside from the hydrogenation treatment step, for example, one or more steps of a step of mixing the crude 1,3-butylene glycol with water and an organic solvent, phase-separating the mixture into a water layer and an organic layer and then obtaining a water layer containing 1,3-butylene glycol (extraction step), a step of distilling water away from the water layer containing crude 1,3-butylene glycol (dehydration and distillation step) and a step of distilling a low-boiling component away from the crude 1,3-butylene glycol (low-boiling fraction distillation step) and preferably includes the low-boiling fraction distillation step. The order of these steps is not particularly limited, but it is preferable to perform the hydrogenation treatment step, the extraction step, the dehydration and distillation step and the low-boiling fraction distillation step in order from the viewpoint of making the effect of the present invention more significant.

Hereinafter, each step will be described. The crude 1,3-butylene glycol in "the crude 1,3-butylene glycol after the hydrogenation treatment step" means 1,3-butylene glycol as an intermediate before the final product is produced.

### (Extraction Step)

The extraction step in the production method of the product 1,3-butylene glycol of the present embodiment is, for example, a step of mixing the crude 1,3-butylene glycol after the hydrogenation treatment step with water and an organic solvent, phase-separating the mixture into a water layer and an organic layer and then obtaining a water layer containing 1,3-butylene glycol. Here, examples of the organic solvent include chain aliphatic hydrocarbons such as hexane and heptane, cycloaliphatic hydrocarbons such as cyclohexane and methylcyclohexane, aromatic hydrocarbons such as toluene and xylene, ethers such as diethyl ether and dibutyl ether, organic chlorides such as methylene chloride and chloroform, esters such as ethyl acetate and butyl acetate and ketones such as methyl isobutyl ketone, and, among these, ketones or cycloaliphatic hydrocarbons are preferable, and methyl isobutyl ketone or methylcyclohexane is more preferable from the viewpoint of impurity removal. These organic solvents may be used singly or two or more organic solvents may be selected, mixed in an arbitrary ratio and used. The amount of the organic solvent used is preferably 10 to 300 parts by mass and more preferably 20 to 200 parts by mass with respect to 100 parts by mass of the crude 1,3-butylene glycol after the hydrogenation treatment step from the viewpoint of the extraction efficiency.

The amount of water used is preferably 20 to 400 parts by mass and more preferably 40 to 200 parts by mass with respect to 100 parts by mass of the crude 1,3-butylene glycol after the hydrogenation treatment step from the viewpoint of the extraction efficiency. The order of adding water and the organic solvent to the crude 1,3-butylene glycol after the hydrogenation treatment step is not particularly limited. The temperature at which water and the organic solvent are mixed with the crude 1,3-butylene glycol after the hydrogenation treatment step is not particularly limited, but is preferably a temperature of 5°C to 80°C and more preferably a temperature of 10°C to 50°C from the viewpoint of the extraction efficiency.

The crude 1,3-butylene glycol after the hydrogenation treatment step, water and the organic solvent can be mixed by, for example, a batch method, a continuous method or the like. Examples of the case of mixing by a batch method include a form in which the crude 1,3-butylene glycol after the hydrogenation treatment step, water and the organic solvent are put into a mixing tank, preferably stirred for 10 seconds to two hours and then preferably placed still for one minute to two hours to be phase-separated and a water layer containing 1,3-butylene glycol is obtained and the like. After the obtained water layer containing 1,3-butylene glycol is phase-separated by further adding and stirring the organic solvent, the operation of obtaining the water layer containing 1,3-butylene glycol may be repeated, and the number of repetitions is preferably once to three times. In this case, the amount of the organic solvent added per operation is preferably 10 to 300 parts by mass with respect to 100 parts by mass of the crude 1,3-butylene glycol after the hydrogenation treatment step.

As a device in the case of mixing by a continuous method, devices that are ordinarily used for continuous extraction or the like, for example, a combination of a mixer and a settler, a spray tower, a packed tower, a tray tower and the like can be used, and, in particular, a packed tower or tray tower having three or more theoretical plates is preferably used.

### (Dehydration and Distillation Step)

The dehydration and distillation step in the production method of a product 1,3-butylene glycol of the present embodiment is a step of distilling water away from the water layer containing 1,3-butylene glycol obtained in the extraction step. Examples of a distillation device that is used in the dehydration and distillation step include a perforated plate tower, a bubble cap tower and a packed tower, and, among these, a packed tower having seven to 40 theoretical plates is preferable. The distillation tower may be one tower, or two or more towers may be used. As the distillation conditions, the pressure at the tower top portion of the distillation tower is preferably 5 to 20 kPa, and the temperature at the tower bottom portion of the distillation tower is preferably 120°C to 160°C and more preferably 135°C to 155°C. Examples of a specific aspect of the dehydration and distillation step include a method in which the water layer containing 1,3-butylene glycol is continuously supplied from the tower top portion of the distillation tower, a distillate containing a large amount of water is continuously extracted from the tower top portion and, simultaneously, a top bottom liquid containing a large amount of 1,3-butylene glycol is continuously extracted from the tower bottom portion.

### (Low-Boiling Fraction Distillation Step)

The low-boiling fraction distillation step in the production method of a product 1,3-butylene glycol of the present embodiment is a step of distilling the low-boiling component away from a liquid containing a large amount of 1,3-butylene glycol obtained in, for example, the hydrogenation treatment step or the dehydration and distillation step. Examples of a distillation device that is used in the low-boiling fraction distillation step include a perforated plate tower, a bubble cap tower and a packed tower, and, among these, a packed tower having seven to 40 theoretical plates is preferable. The distillation tower may be one tower, or two or more towers may be used. As the distillation conditions, the pressure at the tower top portion of the distillation tower is preferably 1 to 20 kPa, and the temperature at the tower bottom portion of the distillation tower is preferably 100°C to 160°C and more preferably 110°C to 140°C. Examples of a specific aspect of the low-boiling fraction distillation step include a method in which a liquid containing a large amount of 1,3-butylene glycol is continuously supplied from the tower top portion of the distillation tower, a distillate containing a large amount of a low-boiling component is continuously extracted from the tower top portion and, simultaneously, a top bottom liquid containing a larger amount of 1,3-butylene glycol is continuously extracted from the tower bottom portion.

### [Product 1,3-Butylene Glycol]

In a product 1,3-butylene glycol of the present embodiment, the area percentage of a peak of 1,3-butylene glycol in a gas chromatography analysis under the following conditions is 99.5% or more, and the weight fraction of a dinitrophenylhydrazine derivative of 6-hydroxy-2-hexanone in an HPLC (High Performance Liquid Chromatography) analysis under the following conditions is 18.0 ppm or less.

### [Conditions of Gas Chromatography Analysis]

Analysis column: a column in which polyethylene glycol is used as a stationary phase (length of 30 m × inner diameter of 0.25 mm × film thickness of 0.25 µm)
Temperature rising conditions: a temperature is raised from 80°C up to 230°C at 5 °C/minute and then retained at 230°C for 10 minutes
Sample introduction temperature: 250°C
Carrier gas: nitrogen
Gas flow rate in column: 0.5 mL/minute
Detector and detection temperature: flame ionization detector (FID), 250°C
Control mode: constant flow
Split ratio: 50:1
Sample injection condition: 1 µL

### [Conditions of HPLC Analysis]

### (Sample Preparation)

1000 µL of a solution of 2,4-dinitrophenylhydrazine which is extracted by adding 5 mL of acetonitrile to a cartridge packed with spherical silica gel coating 2,4-dinitrophenylhydrazine and 100 µL of 0.2 mol/L hydrochloric acid are added to 0.05 g of the product 1,3-butylene glycol and reacted at 45°C for two hours.

### (Analysis Conditions)

Measurement sample: A reaction liquid that is obtained by the sample preparation is diluted to 2 mL with a mobile phase that is used in HPLC, and this diluted liquid is used as a measurement sample.
Detector: UV-Vis detector
Detection wavelength: 369 nm
Analysis column: A column in which palmitamidopropyl group-modified silica gel (particle diameter: 5 µm, inner diameter × length = 4.6 mm × 25 cm, pore size: 100 Å, surface coating level: 2.7 µmol/m², surface area: 450 m²/g, metal impurity: less than 5 ppm, carbon content: 19.5%) is used as a stationary phase
Column temperature: 40°C
Mobile phase: acetonitrile/distilled water = 50/50 (volume ratio)
Mobile phase flow rate: 0.4 mL/min.
Sample injection amount: 20 µL

In the product 1,3-butylene glycol of the present embodiment, the area percentage of a peak of 1,3-butylene glycol in the gas chromatography analysis under the above-described conditions is 99.5% or more, for example, preferably 99.6% or more, more preferably 99.7% or more, still more preferably 99.8% or more and particularly preferably 99.9% or more depending on additionally required product qualities. The "area percentage" of a peak means the percentage of the area of a specific peak with respect to the sum of the areas of all peaks appearing in a chart. In addition, all peaks mean all of the peaks that appear in a case where the analysis is continued until a relative retention time of 2.2 and stopped when the relative retention time of the peak of 1,3-butylene glycol is regarded as 1.0. When the area percentage of the peak is within the above-described range, generation of an odor or skin sensitization tends to be much more reduced.

In the product 1,3-butylene glycol of the present embodiment, the weight fraction of a dinitrophenylhydrazine derivative of 6-hydroxy-2-hexanone in the HPLC analysis under the above-described conditions is 18.0 ppm or less, for example, preferably 16.5 ppm or less, more preferably 10.0 ppm or less, still more preferably 4.0 ppm or less and particularly preferably 2.0 ppm or less depending on additionally required product qualities.

Here, the dinitrophenylhydrazine derivative of 6-hydroxy-2-hexanone is a 2,4-dinitrophenylhydrazine (hereinafter, also referred to as "DNPH")-derivatized compound from 6-hydroxy-2-hexanone or an acetal compound thereof that is contained in 1,3-butylene glycol, and, in an HPLC analysis under specific conditions to be described below, the peaks of the derivative appears within a relative retention time range of 1.7 to 1.9 when the relative retention time of the peak of DNPH is regarded as 1.0. In the HPLC analysis, the area value of the absorbance at 369 nm of the peaks that appear within a relative retention time range of 1.7 to 1.9 when the relative retention time of the peak of 2,4-dinitrophenylhydrazine is regarded as 1.0 is measured with an ultraviolet spectrophotometer.

### [Measurement Conditions of Sniff GC/MS Measurement]

Sniff GC/MS is performed using a device shown in a device schematic view of Figure 1. This device is made up of a concentrator that concentrates the sample vapor of a gas-phase part of a measurement sample, GC that separates the concentrated vapor with a capillary column, a sniffing system capable of directly smelling a separated component and MSD (mass spectrometer detector) that determines the qualities and quantity of the separated component.

### [Skin Sensitization Test]

The product 1,3-butylene glycol in the present embodiment has reduced skin sensitization. Here, skin sensitization refers to causing an allergic reaction after skin contact. For the evaluation of skin sensitization, it had been normal to use experimental animals, but a direct peptide reactivity assay (DPRA), which is an in chemico test, was adopted as an alternative test in OECD guideline TG442C in 2015 from the viewpoint of animal welfare, and in the present embodiment as well, skin sensitization is evaluated by a test in accordance with DPRA. In more detail, skin sensitization is evaluated according to a method described in experimental examples to be described below.

### Examples

Hereinafter, the present invention will be more specifically described with examples, but the present invention is not limited to the following examples. As crude 1,3-butylene glycol that acted as a raw material, 1,3-butylene glycol obtained by hydrogenation reduction of acetaldol and subsequent distillation that removes a low-boiling component and further distillation that removes a high-boiling component was used. A variety of analyses and evaluations were performed according to the following.

### [HPLC Analysis]

Under the following conditions, an HPLC analysis of a product 1,3-butylene glycol was performed.

### (Conditions of HPLC Analysis)

Sample preparation: 1000 µL of a solution from which 2,4-dinitrophenylhydrazine had been extracted by adding 5 mL of acetonitrile to a 2,4-dinitrophenylhydrazine cartridge (InertSep mini AERO DNPH, GL Sciences Inc.) and 100 µL of 0.2 mol/L hydrochloric acid were added to 0.05 g of 1,3-butylene glycol and reacted at 45°C for two hours. The reaction liquid was diluted to 2 mL with a mobile phase that was used in HPLC, and the solution was used as a measurement sample.
Analysis device: Agilent 1200 Series manufactured by Agilent Technologies, Inc.
Detector: Agilent 1200 Series UV-Vis detector G1314B manufactured by Agilent Technologies, Inc.
Detection wavelength: 369 nm
Analysis column: SUPELCO (R) Ascentis (R) RP-Amide (particle diameter: 5 µm, inner diameter × length = 4.6 mm × 25 cm) manufactured by Merck KGaA
Analysis condition: Column temperature of 40°C
Mobile phase: Acetonitrile/distilled water = 50/50 (volume ratio)
Mobile phase flow rate: 0.4 mL/min.
Sample injection condition: 20 µL
Calibration curve: Created using a separately synthesized dinitrophenylhydrazine derivative of 6-hydroxy-2-hexanone
SUPELCO (R) Ascentis (R) RP-Amide manufactured by Merck KGaA is a column in which palmitamidopropyl group-modified silica gel (particle diameter: 5 µm, inner diameter × length = 4.6 mm × 25 cm, pore size: 100 Å, surface coating level: 2.7 µmol/m², surface area: 450 m²/g, metal impurity: less than 5 ppm, carbon content: 19.5%) is used as a stationary phase.

In the measurement of the sample prepared by the above-described method, the absorbance at 369 nm of the peaks of the dinitrophenylhydrazine derivative of 6-hydroxy-2-hexanone was measured with an ultraviolet spectrophotometer. The obtained absorbance was converted to the weight fraction of the dinitrophenylhydrazine derivative of 6-hydroxy-2-hexanone in 1,3-butylene glycol using "peak weight fraction (ppm) = 0.220 × absorbance", which was a formula derived with the calibration curve. When the relative retention time of the peak of DNPH was regarded as 1.0, peaks that appeared within a relative retention time range of 1.7 to 1.9 were regarded as the peaks of the dinitrophenylhydrazine derivative of 6-hydroxy-2-hexanone.

### [Gas Chromatography Analysis]

Under the following conditions, a gas chromatography analysis of the product 1,3-butylene glycol, which was a subject, was performed.

### (Conditions of Gas Chromatography Analysis)

Analysis device: 7890B gas chromatography system manufactured by Agilent Technologies, Inc.
Analysis column: DB-WAX (length 30 m × inner diameter 0.25 mm × film thickness 0.25 µm) manufactured by Agilent Technologies, Inc.
Temperature rising conditions: The temperature was raised from 80°C up to 230°C at 5 °C/minute and then retained at 230°C for 10 minutes.
Sample introduction temperature: 250°C
Carrier gas: Nitrogen
Gas flow rate in column: 0.5 mL/minute
Detector and detection temperature: Flame ionization detector (FID), 250°C
Control mode: Constant flow
Split ratio: 50:1
Sample injection condition: 1 µL

### [Skin Sensitization Test]

Regarding 1,3-butylene glycol obtained in Examples 1 and 2 and Comparative Examples 1 and 2, skin sensitization was evaluated according to the following method.

### (Conditions of Skin Sensitization Test)

15 mg of Cysteine-containing peptide for DPRA manufactured by Scrum Inc. and 30 mL of a 0.05 M phosphate buffer were mixed together to prepare a 0.05 M phosphate buffer solution containing 0.667 mM of the peptide (hereinafter, peptide solution). After one hour elapsed from the preparation of the peptide solution, 750 µL of the peptide solution and 250 µL of acetonitrile were put into each of three brown sample bottles for HPLC to prepare three reference solutions. After two hours elapsed from the preparation of the reference solutions, 750 µL of the peptide solution, 200 µL of acetonitrile and 50 µL of 1,3-butylene glycol were put into a different brown sample bottle for HPLC to prepare a sample solution (sample solution 1). Furthermore, sample solutions were prepared by the same operation after four hours and six hours elapsed from the preparation of the sample solution, respectively (sample solution 2 and sample solution 3). Regarding each of the three reference solutions and the sample solutions (sample solution 1, sample solution 2 and sample solution 3), measurement by HPLC analysis was performed after 72 ± 2 hours elapsed from the preparation of each solution, and the average values of three measurements were calculated for the peak height of the peptide in the reference solutions and the sample solutions, respectively. The peptide decrease percentage was calculated using the following formula (1) from the calculated average values of three measurements.

Peptide decrease percentage (%) = (average value of three measurements for peak height of peptide in sample solutions/average value of three measurements for peak height of peptide in reference solutions) × 100 (1)

In addition, regarding the present test, samples of the product 1,3-butylene glycol to be compared were tested on the same day from the viewpoint of the reproducibility.

### (Conditions of HPLC Analysis in Skin Sensitization Test)

Analysis device: Agilent 1260 Infinity II manufactured by Agilent Technologies, Inc.
Detector: UV-Vis detector G7114A in Agilent 1260 Infinity II manufactured by Agilent Technologies, Inc.
Detection wavelength: 220 nm
Analysis column: Zorbax SB-C-18 (particle diameter 3.5 µm, inner diameter × length = 2.1 mm × 10 mm) manufactured by Agilent Technologies, Inc.
Column temperature: 30°C
Measurement time: 20 min.
Mobile phase:
   A 0.1 vol% of Trifluoroacetic acid aqueous solution
   B 0.085 vol% of Trifluoroacetic acid acetonitrile solution
   Gradient:
      A/B = 90/10 to 75/25 (10 min.)
      A/B = 75/25 to 10/90 (1 min.)
      A/B = 10/90 (2 min.)
      A/B = 10/90 to 90/10 (0.5 min.)
      A/B = 90/10 (6.5 min.)
      Mobile phase flow rate: 0.35 mL/min.
      Sample injection amount: 5 µL

### [GC/MS-Olfactometry Analysis (Gas Chromatography Mass Analysis with Concentrator)]

Methyl vinyl ketone that was contained in the product 1,3-butylene glycol obtained in each of Examples 1 and 2 and Comparative Examples 1 and 2 was measured by GC/MS-olfactometry analysis according to the following method. The GC/MS-olfactometry was performed using a device shown in a device schematic view of Figure 1. This device is made up of a concentrator that concentrates the sample vapor of a gas-phase part of a measurement sample, GC that separates the concentrated vapor with a capillary column, a sniffing system capable of directly smelling a separated component and MSD (mass spectrometer detector) that determines the qualities and quantity of the separated component.

### (Pretreatment)

The product 1,3-butylene glycol was distilled at an oil bath temperature of 120°C and 1.2 kPa with a distillation device equipped with a 500 mm-high packed tower (inner diameter 22 mm) packed with 3 mm-size dixon packing in a manner that the packing height became 425 mm, and 1,3-butylene glycol containing a low-boiling component as much as a weight fraction of 1% of the amount of liquid charged from the distillation device top portion was distilled away. 1,3-Butylene glycol obtained as a still residue of the distillation device was used as a measurement sample of the GC/MS-olfactometry analysis.

### (Concentrator)

Concentrator: Entech 7200 automated concentrator manufactured by Entech Instruments, Inc.
Sample amount: 5 g
Injection amount: 200 mL of a gas-phase part that had been placed still at 30°C for 20 minutes or longer
(separately, 100 mL of an internal standard substance was injected aside from the gas-phase part)
Internal standard substance: 100 mL (heavy toluene standard gas: concentration 10 vol ppb, dilution gas was nitrogen, Sumitomo Seika Chemicals Company, Limited.)
Concentration method: CTD mode (Cold Trap Dehydration)
Temperature condition of dehydration module 1 (empty trap): Trap temp. -40°C, desorption temp. 0°C
Temperature condition of Cold Tenax (R) Module 2 (Tenax Trap): trap temp. -30°C, desorption temp. 200°C
Temperature condition of focusing module 3 (cryo focus): Trap temp. -165°C, desorption temp. 100°C
Sample flow rate: 50 mL/min.
He flush volume: 75 mL
M1 to M2 volume: 40 mL (100 mL/min.)
M2 to M3 time: 3.0 min.
Injection time: 0.3 min.

### (GC)

Measuring equipment: Agilent 7890B gas chromatography system manufactured by Agilent Technologies, Inc.
Analysis column: DB-1 manufactured by Agilent Technologies, Inc. (a column in which dimethyl polysiloxane was used as a stationary phase; length 60 m × inner diameter 320 µm × film thickness 1 µm)
Temperature rising program: 35°C (2 min.) → 10°C/min. -> 240°C (7.5 min.)
Carrier gas: Helium
Control mode: Constant pressure (153.09 kPa)

The concentrated sample was separated with a capillary column and then sent to ODP3 (sniffing system) and MSD 5977B (mass spectrometer detector) manufactured by GESTEL in a ratio of 1/1.

### (MDS: Mass spectrometer detector)

Detector: Agilent 5977B MSD manufactured by Agilent Technologies, Inc.
Ionization mode: EI
Measurement type: Scan
Ion source temperature: 250°C
Quadrupole temperature: 150°C
Electron energy: 70.0 eV
Scan start mass: 30
Scan end mass: 400

Calibration curve: Created using heavy toluene standard gas (concentration: 10 vol ppb, dilution gas: nitrogen) manufactured by Sumitomo Seika Chemicals Company, Limited.

During data analysis, the EIC peak area (EIC: m/z 55.000) of methyl vinyl ketone that appeared at relative retention times of 0.59 to 0.61 when the relative retention time of heavy toluene was regarded as 1.0 was confirmed using an extracted ion chromatogram (EIC). The heavy toluene-equivalent concentration of methyl vinyl ketone in the vapor of 1,3-butylene glycol was calculated using "heavy toluene-equivalent concentration (vol ppb) = 3.15 × 10⁻⁶ × EIC peak area", which is a formula derived using the calibration curve.

### [Example 1]

The crude 1,3-butylene glycol and a hydrogen gas were circulated in a catalyst layer packed with 10 mL of a 0.5% Pd/C catalyst so that LHSV became 8.0 h⁻¹ and GHSV became 420 h⁻¹ to perform hydrogen reduction (hydrogenation treatment). The hydrogen pressure during the reaction was 0.7 MPa, and the reaction temperature was set to 100°C.

As a result of HPLC analysis of the obtained product 1,3-butylene glycol, the weight fraction of a dinitrophenylhydrazine derivative of 6-hydroxy-2-hexanone was 9.9 ppm. In addition, as a result of gas chromatography analysis under the above-described conditions, the area percentage of the peak of 1,3-butylene glycol in the gas chromatography analysis was 99.8%.

As a result of a skin sensitization test for the product 1,3-butylene glycol, the relative value thereof with respect to the peptide decrease percentage of Comparative Example 1 that was regarded as 100 was 73.

As a result of GC/MS-olfactometry analysis of the product 1,3-butylene glycol, the heavy toluene-equivalent concentration of methyl vinyl ketone was 0.21 vol ppb.

These results regarding the product 1,3-butylene glycol are shown in Table 1 and Table 2.

### [Example 2]

The same operation as in Example 1 was performed except that the reaction temperature in the hydrogen reduction was set to 70°C. As a result of HPLC analysis of the obtained product 1,3-butylene glycol, the weight fraction of a dinitrophenylhydrazine derivative of 6-hydroxy-2-hexanone was 16.3 ppm. In addition, as a result of gas chromatography analysis under the above-described conditions, the area percentage of the peak of 1,3-butylene glycol in the gas chromatography analysis was 99.8%.

As a result of a skin sensitization test for the product 1,3-butylene glycol, the relative value thereof with respect to the peptide decrease percentage of Comparative Example 1 that was regarded as 100 was 85.

As a result of GC/MS-olfactometry analysis on the product 1,3-butylene glycol, the heavy toluene-equivalent concentration of methyl vinyl ketone was 0.25 vol ppb.

These results regarding the product 1,3-butylene glycol are shown in Table 1 and Table 2.

### [Comparative Example 1]

The same operation as in Example 1 was performed except that NiSAT340 (composition; NiO, SiCt, Al₂O₃ and the like) manufactured by Clariant AG was used as the catalyst in the hydrogen reduction. As a result of HPLC analysis of the obtained product 1,3-butylene glycol, the weight fraction of a dinitrophenylhydrazine derivative of 6-hydroxy-2-hexanone was 20.5 ppm. In addition, as a result of gas chromatography analysis under the above-described conditions, the area percentage of the peak of 1,3-butylene glycol in the gas chromatography analysis was 99.7%.

A skin sensitization test was performed on the product 1,3-butylene glycol, and the obtained peptide decrease percentage was regarded as 100 to use this peptide decrease percentage as a reference at the time of relatively comparing those of other examples and comparative example.

As a result of GC/MS-olfactometry analysis of the product 1,3-butylene glycol, the heavy toluene-equivalent concentration of methyl vinyl ketone was 2.2 vol ppb.

These results regarding the product 1,3-butylene glycol are shown in Table 1 and Table 2.

### [Comparative Example 2]

The same operation as in Comparative Example 1 was performed except that the reaction temperature in the hydrogen reduction was set to 70°C. As a result of HPLC analysis of the obtained product 1,3-butylene glycol, the weight fraction of a dinitrophenylhydrazine derivative of 6-hydroxy-2-hexanone was 23.3 ppm. In addition, as a result of gas chromatography analysis under the above-described conditions, the area percentage of the peak of 1,3-butylene glycol in the gas chromatography analysis was 99.8%.

As a result of a skin sensitization test for the product 1,3-butylene glycol, the relative value thereof with respect to the peptide decrease percentage of Comparative Example 1 that was regarded as 100 was 121.

As a result of GC/MS-olfactometry analysis of the product 1,3-butylene glycol, the heavy toluene-equivalent concentration of methyl vinyl ketone was 0.28 vol ppb.

These results regarding the product 1,3-butylene glycol are shown in Table 1 and Table 2.

**[Table 1]**

| | | Example 1 | Example 2 | Comparative Example 1 | Comparative Example 2 |
|---|---|---|---|---|---|
| HPLC analysis | 6H2H-DNPH (ppm) * | 9.9 | 16.3 | 20.5 | 23.3 |
| Skin sensitization test | Relative value of peptide decrease percentage | 73 | 85 | 100 | 121 |

| | | | | | |
|---|---|---|---|---|---|
| * Weight fraction of 6H2H-DNPH (dinitrophenylhydrazine derivative of 6-hydroxy-2-hexanone) | | | | | |

**[Table 2]**

| | | Example 1 | Example 2 | Comparative Example 1 | Comparative Example 2 |
|---|---|---|---|---|---|
| GC/MS-olfactometry analysis | MVK (vol ppb) * | 0.21 | 0.25 | 2.2 | 0.28 |

| | | | | | |
|---|---|---|---|---|---|
| * Heavy toluene-equivalent concentration of MVK (methyl vinyl ketone) | | | | | |

### Industrial Applicability

1,3-Butylene glycol of the present invention is industrially available as a raw material of synthetic resins, a raw material of surfactants, a solvent, an antifreeze, a cosmetic raw material or the like.

## Claims

1. A method for producing a product 1,3-butylene glycol, comprising: a step of subjecting crude 1,3-butylene glycol to hydrogenation treatment using a Pd/C catalyst.

2. The method for producing the product 1,3-butylene glycol according to claim 1, wherein in the step of subjecting crude 1,3-butylene glycol to hydrogenation treatment, a reaction temperature is from 50°C to 120°C.

3. The method for producing the product 1,3-butylene glycol according to claim 1 or 2, wherein in the step of subjecting crude 1,3-butylene glycol to hydrogenation treatment, a content of 6-hydroxy-2-hexanone in the crude 1,3-butylene glycol is reduced.

4. A product 1,3-butylene glycol, wherein an area percentage of a peak of 1,3-butylene glycol in a gas chromatography analysis under the following conditions is 99.5% or more, and a weight fraction of a dinitrophenylhydrazine derivative of 6-hydroxy-2-hexanone in an HPLC analysis under the following conditions is 18.0 ppm or less,
wherein the conditions of the gas chromatography analysis are as follows:
analysis column: a column in which polyethylene glycol is used as a stationary phase (length of 30 m × inner diameter of 0.25 mm × film thickness of 0.25 µm),
temperature rising conditions: a temperature is raised from 80°C up to 230°C at 5 °C/minute and then retained at 230°C for 10 minutes,
sample introduction temperature: 250°C,
carrier gas: nitrogen,
gas flow rate in column: 0.5 mL/minute,
detector and detection temperature: flame ionization detector (FID) and 250°C,
control mode: constant flow
split ratio: 50:1,
sample injection condition: 1 µL, and
wherein the conditions of the HPLC analysis are as follows:
the sample preparation is as follows:
1000 µL of a solution of 2,4-dinitrophenylhydrazine, which is extracted by adding 5 mL of acetonitrile to a cartridge packed with spherical silica gel coated with 2,4-dinitrophenylhydrazine, and 100 µL of 0.2 mol/L hydrochloric acid are added to 0.05 g of the product 1,3-butylene glycol, and a resultant mixture is reacted at 45°C for two hours.
(Analysis Conditions)
Measurement sample: a reaction liquid obtained by the sample preparation is diluted to 2 mL with a mobile phase that is used in HPLC, and the diluted liquid is used as a measurement sample,
Detector: UV-Vis detector,
Detection wavelength: 369 nm,
Analysis column: a column in which palmitamidopropyl group-modified silica gel (particle diameter: 5 µm, inner diameter × length = 4.6 mm × 25 cm, pore size: 100 Å, surface coating level: 2.7 µmol/m², surface area: 450 m²/g, metal impurity: less than 5 ppm, carbon content: 19.5%) is used as a stationary phase,
Column temperature: 40°C,
Mobile phase: acetonitrile/distilled water = 50/50 (volume ratio),
Mobile phase flow rate: 0.4 mL/min.,
Sample injection amount: 20 µL
